Europäisches Patentamt

European Patent Office

Office européen des brevets

(19)

(11) Publication number: **0 069 514**
**A2**

(12) # EUROPEAN PATENT APPLICATION

(21) Application number: 82303315.4

(22) Date of filing: 25.06.82

(51) Int. Cl.³: **B 01 J 23/74**
**C 07 C 51/12**

(30) Priority: 26.06.81 JP 98286/81

(43) Date of publication of application:
12.01.83 Bulletin 83/2

(84) Designated Contracting States:
FR GB IT

(71) Applicant: TOYO ENGINEERING CORPORATION
2-5, Kasumigaseki 3-chome
Chiyoda-ku, Tokyo 100(JP)

(71) Applicant: Tominaga, Hiroo
444-53, Kogane
Matsudo-Shi Chiba(JP)

(72) Inventor: Fujimoto, Kaoru
6-18-1-1031, Minamiohi Shinagawa-ku
Tokyo(JP)

(72) Inventor: Shikada, Tsutomu
3-5-4-305, Higashi Ayase Adachi-ku
Tokyo(JP)

(72) Inventor: Tominaga, Hiroo
444-53, Kogane
Matsudo-shi Chiba(JP)

(74) Representative: Shipton, Gordon Owen et al,
W.P. THOMPSON & CO. Coopers Building Church Street
Liverpool L1 3AB(GB)

(54) Alcohol carbonylation catalysts.

(57) An alcohol carbonylation catalyst which comprises
nickel, cobalt or a compound thereof carried on a carbo-
naceous carrier such as active carbon, carbon black, coke or
an inorganic substance having a carbonaceous deposit. With
this carbonylation catalyst, problems of conventional proces-
ses such as corrosion of the reactor and the formation of
volatile, poisonous nickel carbonyl are avoided.

EP 0 069 514 A2

1.

## DESCRIPTION

## "ALCOHOL CARBONYLATION CATALYSTS"

The present invention relates to alcohol carbonylation catalysts. More particularly, the present invention relates to highly valuable solid catalysts used for the production of monocarboxylic acids or their esters by reacting an alcohol with carbon monoxide.

For the production of a monocarboxylic acid or its ester by the carbonylation reaction of an alcohol with carbon monoxide, there has been employed broadly and practically a process wherein the reaction is carried out in the liquid phase in the presence of a cobalt compound such as cobalt acetate as a catalyst and an iodine compound such as potassium iodide or methyl iodide as a co-catalyst.

However, this process must be carried out under severe reaction conditions comprising a temperature of 210 to 250°C and a pressure of 490 to 700 atm in order to maintain a stable, sufficient catalytic activity, since it is considered that the catalyst must be present in the form of a metal carbonyl under the reaction conditions. Further, according to this process, the reaction selectivity is low and, therefore, an aldehyde, carbon dioxide and hydrogen are by-produced and the ultimate yield of acetic acid in a commercial plant is as low as about 59 % based on carbon monoxide or about 87 % based on methanol. Another disadvantage of this process is that a reaction vessel made of an expensive anti-corrosive material must be used, since the liquid reaction system contains corrosive compounds such as acetic acid and iodides.

Recently, there has been developed a process for the carbonylation of methanol wherein a rhodium complex is used as a catalyst and methyl iodide or hydrogen iodide is used as a co-catalyst (see the specification of Japanese Patent Publication No.

2.

334/1972). According to this process, the reaction proceeds under mild conditions comprising a temperature of 175 to 245°C and a pressure of 10 to 30 atm and yield of acetic acid is as high as 90 % based on carbon monoxide or 99 % based on methanol.

However, this process has a serious problem that remarkably expensive rhodium, of which availability on the market is limited because of its poor natural resources, must be used as the catalyst. In addition, a problem of the corrosion of the apparatus is unavoidable, since an iodide is used in liquid phase.

There have been proposed processes for the carbonylation of methanol using a nickel catalyst in liquid phase. For example, there have been proposed processes wherein the carbonylation reaction of methanol is carried out in the presence of an organotin compound (Japanese Patent Laid-Open No. 59615 /1978), an organic nitrogen compound (Japanese Patent Laid-Open No. 59211/ 1979) or an organophosphorus (III) compound (Japanese Patent Laid-Open No. 66614/1979) in addition to a nickel catalyst under a carbon monoxide pressure of up to 50 atm.

However, the above-mentioned processes wherein the nickel catalyst is used in the liquid phase is economically disadvantageous, since an expensive iodine compound used as a co-catalyst is required in a large amount (more than 20 molar % based on methanol). Further, in these processes, the reactor is corroded and volatile, virulently poisonous nickel carbonyl is formed to pose a problem of operation safety.

The inventors made intensive investigations for the purposes of overcoming the defects of the above-mentioned, conventional techniques and developing an. effective catalyst which is free from the problems of

3.

corrosion of the reaction apparatus and operation safety and which facilitates the operation. As a result, the inventors have found that a catalyst comprising an active ingredient such as nickel supported on a carbonaceous carrier such as activated carbon or carbon black meets the purposes and that if such a catalyst is used, the carbonylation of an alcohol proceeds efficiently in the gas phase and the above-mentioned problems of corrosion of the reaction apparatus and operation safety can be solved. The present invention has been completed on the basis of these findings. The present invention provides an alcohol carbonylation catalyst which comprises nickel, cobalt or a compound thereof supported on a carbonaceous carrier.

The carrier of the catalyst of the present invention must be carbonaceous. Particular examples of the carrier are activated carbon, carbon black and coke. In addition, inorganic carriers such as silica, alumina, silica-alumina or zeolite on which carbon has been deposited or on which an active ingredient has been carried and then carbon has been deposited may also be used. If a substance other than the carbonaceous substance is used as the catalyst carrier, the ultimate yield of acetic acid is so small that the object of the present invention cannot be attained.

As the active ingredient to be carried by the above-mentioned catalyst carrier, there may be used nickel, cobalt or a compound thereof. Among them, nickel is particularly preferred. It is supposed that this active ingredient is converted into a partially carbonylated metal compound in the reaction and, therefore, the starting material may be a metal or metal compound. The active ingredient-carrying rate of the catalyst is not particularly limited and it may be determined properly. Generally, the carrying

4.

rate is in the range of 0.1 to 50 wt. %, preferably 0.5 to 20 wt. %.

The carbonylation of an alcohol in the presence of the catalyst of the present invention comprising the above-mentioned carrier and active ingredient is carried out generally under the following conditions; said catalyst and a volatile halogen compound such as an iodine compound or bromine compound as a co-catalyst are added to the reaction system. The co-catalyst is preferably an alkyl iodide such as methyl iodide or an alkyl bromide such as methyl bromide. The amount of the co-catalyst is not particularly limite and is generally in the range of 0.1 to 50 mol, preferably 1 to 20 mol, per 100 mol of the alcohol. Further, a promotor may be added to the reaction system to enhance the reaction velocity. As preferred promotors, there may be mentioned metals such as alkali metals, iron, copper, silver, molybdenum, chromium, tungsten, tin and manganese as well as their compounds.

The ratio of the alcohol to carbon monoxide in the feed to the reactor can be stoichiometric. However, the ratio is not always limited to the stochiometric ratio but either of them may be used in excess. Generally, the molar ratio of the alcohol to carbon monoxide is in the range of 100/1 to 1/100, preferably 10/1 to 1/10. It is unnecessary to use pure carbon monoxide. Carbon monoxide having some impurities may also be used. Water as an impurity in carbon monoxide causes no problem in the reaction. Further, the catalytic activity is not affected by small amount of hydrogen.

However, the presence of a large amount of hydrogen is undesirable, since the amount of methane by-produced is increased. The alcohols used as the starting material include, for example, methanol, ethanol, propanol, butanol or benzyl alcohol. Among

5.

them, methanol or benzyl alcohol is particularly preferred. A reason therefor is that if methanol or benzyl alcohol is used, the dehydration reaction does not occur in the course of the carbonylation reaction and, therefore, the intended monocarboxylic acid or its ester is obtained in a quite high yield.

It is desirable that the reaction effected in the presence of the catalyst of the present invention is carried out under such conditions that both starting materials and product are in the gaseous state. However, the reaction proceeds without any trouble even if a high boiling material such as an alcohol or monocarboxylic acid is present in liquid form. The reaction temperature should be 100 to 400°C, preferably 150 to 350°C. The reaction pressure should be 0.1 to 300 atm, preferably 1 to 100 atm.

The catalyst of the present invention has the following merits: (1) it is a solid catalyst which is advantageous from the viewpoint of the mechanism and which can be handled easily because of its physical and chemical properties, (2) the active ingredient is not an expensive noble metal such as rhodium but is an inexpensive base metal, and (3) the catalyst is usable also in a fluidized bed type reactor in which the temperature control and heat removal are easy. Further, if the carbonylation reaction is carried out in the presence of this catalyst, an excellent productivity can be obtained at a relatively low temperature under a low pressure and an intended mono-carboxylic acid or its ester (which per se is useful and is easily convertible into the monocarboxylic acid and alcohol) can be obtained with a high selectivity. In addition, since the reaction carried out in the presence of the catalyst of the present invention proceeds in the gas phase, the reaction apparatus is

6.

virtually not corroded and even if by-products are formed, they can be separated out of the intended product and easily disposed of.

As described above in detail, the catalyst of the present invention can be used broadly in the chemical field as an extremely effective catalyst for the production of a monocarboxylic acid from an alcohol.

The following examples will further illustrate the present invention.

Example 1

A commercially available activated carbon (Shirasagi C; product of Takeda Yakuhin Kogyo Co., Ltd.) was pulverized and 20- to 40-mesh fraction was collected. 100 g of the fraction was added to an aqueous nickel nitrate solution containing 2.5 g of metallic nickel. After the evaporation of water to dryness, the material was further dried at 120°C for 24 h and then heat-treated at 400°C in a nitrogen stream for 2 h. It was subsequently subjected to the reduction in hydrogen at 400°C for 2 h to obtain a catalyst. A mixture of methanol, methyl iodide and carbon monoxide in a ratio shown below was fed over the catalyst to form acetic acid by the carbonylation of methanol. The results are shown in Table 1.

A fixed-bed pressure flow reactor was used for the reaction and the reaction condition comprised a reaction temperature as shown in Table 1, a molar ratio of feeding of methanol : methyl iodide : carbon monoxide of 20:1:19, a total pressure ot 10 $kg/cm^2$G and a contact time W/F of 10 g.cat.h/mol.

Example 2

The same activated carbon as in Example 1 was impregnated with an aqueous solution of nickel acetate, nickel chloride, nickel acetylacetate or nickel iodide. After the evaporation of water to dryness, the material was further dried at 120°C for 24 h to obtain catalysts. The carbonylation of methanol was carried out in the presence of these. catalysts under the same reaction conditions as in Example 1. The results are shown in Table 1.

Example 3

A catalyst was obtained in the same manner as in Example 1 except that an inorganic carrier having carbon black, coke or carbon deposited thereon was used. The carbonylation of methanol was carried out in the presence of this catalyst under the same conditions as in Example 1. The results are shown in Table 1.

Example 4

The carbonylation of methanol was carried out in the presence of the catalyst prepared in Example 1 under reaction conditions comprising a reaction temperature of 202 to 345°C, a molar ratio of methyl iodide to methanol of 0.01 to 0.10, a molar ratio of carbon monoxide to (methanol + methyl iodide) of 1/1, total pressure of 0 to 14 $kg/cm^2G$ and W/F of 2.5 to 20 g.cat.h/mol. The results are shown in Table 2.

Example 5

The carbonylation of methanol was carried out in the presence of the catalyst prepared in Example 1 under the same reaction conditions as in Example 1 except that methyl iodide was replaced with methyl bromide. The results are shown in Table 1.

Example 6

The same activated carbon as in Example 1 was impregnated with an aqueous cobalt nitrate solution or aqueous ferric nitrate solution (containing the salt in an amount corresponding to 2.5 parts by weight of the metal per 100 parts by weight of the carrier) and it was then treated in the same manner as in Example 1 to obtain a catalyst. The carbonylation reaction of methanol was carried out under the same reaction conditions as in Example 1. The results are shown in Table 1.

Example 7

The carbonylation of methanol was carried out under the same reaction conditions as in Example 1 except that the catalyst prepared in Example 6 was used and that methyl iodide was replaced with methyl bromide. The results are shown in Table 1.

Example 8

The carbonylation of ethanol was carried out in the presence of the catalyst prepared in Example 1 at a reaction temperature of 249°C under the same reaction conditions as in Example 1 except that methanol was replaced with ethanol. As a result, 22.8% of starting ehtanol was converted into ethyl propionate and 0.8 % thereof was converted into propionic acid.

Comparative Example 1

A catalyst was prepared in the same manner as in Example 1 except that a commercially available γ-alumina (TKS 99651; a product of Tokai Konetsu Co.) was used as the carrier. The carbonylation of methanol was carried out in the presence of this catalyst under the same conditions as in Example 1. The results are shown in Table 1.

Comparative Example 2

A catalyst was prepared in the same manner as in Example 1 except that a commercially available silica gel (ID Gel; a product of Fuji Davison Chemical Co.) was used as the carrier. The carbonylation of methanol

9.

was carried out in the presence of this catalyst under the same conditions as in Example 1. The results are shown in Table 1.

Table 1

| Ex. No. | Catalyst | | Promotor | Temp. (°C) | Conversion (%) | | Yield (%) | | | |
|---|---|---|---|---|---|---|---|---|---|---|
| | Starting active ingredient | Carrier | | | Meth-anol | Car-bone mono-xide | Meth-ane | Dimethyl-ether | Methyl acetate | Acetic acid |
| 1 | Nickel nitrate | Activated carbon | Methyl iodide | 300 | 99.6 | 71.5 | 3.0 | 2.1 | 21.6 | 69.1 |
| 2 | " | " | " | 257 | 85.1 | 29.2 | 1.4 | 20.8 | 54.8 | 3.2 |
| 2 | Nickel acetate | " | " | 257 | 82.8 | 31.0 | 1.7 | 14.9 | 58.? | 8.0 |
| 2 | Nickel iodide | " | " | 255 | 72.4 | 15.0 | 0.9 | 20.8 | 35.6 | 6.2 |
| 2 | Nickel bromide | " | " | 256 | 83.0 | 28.6 | 1.1 | 14.1 | 48.2 | 2.3 |
| 2 | Nickel chloride | " | " | 257 | 82.0 | 28.1 | 1.5 | 15.8 | 43.1 | 6.3 |
| 2 | Nickel acety-lacetonate | " | " | 257 | 82.5 | 28.6 | 1.3 | 14.2 | 44.8 | 5.9 |
| 3 | Nickel acetate | Carbon black | " | 257 | 80.3 | 30.7 | 1.1 | 15.0 | 50.9 | 7.7 |
| 3 | " | Coke | " | 257 | 42.3 | 14.4 | 1.6 | 8.0 | 28.3 | 3.8 |
| 3 | " | Alumina containing carbon deposited | " | 257 | 39.1 | 12.9 | 1.5 | 6.9 | 25.4 | 3.1 |
| 5 | Nickel nitrate | Activated carbon | Methyl bromide | 300 | 76.4 | 15.1 | 4.2 | 23.6 | 24.2 | 4.1 |
| 6 | Cobalt nitrate | " | Methyl iodide | 292 | 91.8 | 18.2 | 3.1 | 77.7 | 10.8 | 0.4 |
| Comp. Ex. | | | | | | | | | | |
| 1 | Nickel nitrate | Alumina | Methyl iodide | 293 | 93.8 | 4.9 | 0.7 | 96.2 | 2.2 | 0.0 |
| 2 | " | Silica gel | " | 290 | 10.8 | 1.2 | 0.4 | 0.4 | 1.1 | 0.0 |

10.

Table 2

| Ex. | Temp. (°C) | Pressure (kg/cm$^2$·G) | Contact time (g·cat·h/mol) | Methyl iodide conc. (mol %) | Conversion(%) | | Yield (%) | | | |
|-----|-----|-----|-----|-----|-----|-----|-----|-----|-----|-----|
| | | | | | Methanol | Carbon monoxide | Methane | Dimethyl ether | Methyl acetate | Acetic acid |
| 4 | 202 | 10 | 5 | 2.5 | 36.6 | 11.8 | 0.2 | .23.4 | 17.8 | 0.5 |
| 4 | 260 | " | " | " | 66.3 | 32.1 | 1.8 | 15.6 | 48.0 | 3.3 |
| 4 | 320 | " | " | " | 92.3 | 42.1 | 6.8 | 9.4 | 45.2 | 17.8 |
| 4 | 345 | " | " | " | 96.8 | 49.1 | 14.2 | 6.0 | 31.6 | 40.4 |
| 4 | 320 | 0 | 5 | 2.5 | 58.6 | 18.0 | 1.2 | 1.4 | 17.9 | 6.1 |
| 4 | " | 5 | " | " | 82.8 | 38.0 | 5.7 | 7.4 | 53.0 | 14.6 |
| 4 | " | 10 | " | " | 92.3 | 42.1 | 6.8 | 9.4 | 45.2 | 17.8 |
| 4 | " | 14 | " | " | 94.6 | .43.6 | 7.6 | 12.1 | 40.4 | 24.3 |
| 4 | 320 | 10 | 2.5 | 2.5 | 84.7 | 41.5 | 5.3 | 5.3 | 57.1 | 13.9 |
| 4 | " | " | 5 | " | 92.3 | 42.1 | 6.8 | 9.4 | 45.2 | 17.8 |
| 4 | " | " | 10 | " | 99.5 | 53.8 | 17.2 | 5.1 | 24.2 | 52.3 |
| 4 | " | " | 20 | " | 100 | 57.9 | 26.1 | 0.0 | 3.1 | 55.7 |
| 4 | 320 | 10 | 5 | 0.5 | 66.5 | 30.3 | 5.6 | 3.2 | 47.7 | 3.6 |
| 4 | " | " | " | 2.5 | 92.3 | 42.1 | 6.8 | 9.4 | 45.2 | 17.8 |
| 4 | " | " | " | 5.0 | 98.0 | 51.4 | 9.9 | 10.1 | 40.3 | 52.8 |

11.

12.

## CLAIMS

1.    A catalyst, suitable for the alcohol carbonylation process, which comprises a carbonaceous carrier and a substance selected from nickel, cobalt and compounds thereof, supported on said carrier.

2.    A catalyst as claimed in claim 1 wherein said carbonaceous carrier is selected from activated carbon, carbon black, coke and inorganic substances having carbon deposited thereon.

3.    A catalyst as claimed in claim 1 or 2 wherein said substance is supported in an amount of 0.1 to 50 percent by weight.

4.    A process for manufacturing a monocarboxylic acid or an ester thereof which comprises reacting an alcohol and carbon monoxide in the presence of a catalyst as claimed in any one of claims 1 to 3.

5.    A process as claimed in claim 4 in which the reaction is carried out also in the presence of a promotor.